# EUROPEAN PATENT APPLICATION

(11) **EP 1 632 225 A1**
(43) Date of publication of application: **08.03.2006**
(21) Application number: 04746019.1
(22) Date of filing: 10.06.2004
(51) Int. Cl.: A61K 9/14

(54) **DRUG NANO-PARTICLE, METHOD AND APPARATUS FOR PREPARING PHARMACEUTICAL PREPARATION USING THE PARTICLE**

(30) Priority: 11.06.2003 JP 2003166931
(71) Applicant: Nara Machinery Co., Ltd., Ohta-ku, Tokyo 143-0002 (JP)
(72) Inventor: NAGARE, Sanshiro c/o NARA MACHINERY CO. ,LTD., Tokyo 143-0002 (JP); SENNA, Mamoru, Tokyo 182-0035 (JP)
(74) Representative: Kramer - Barske - Schmidtchen
(86) International application number: PCT/JP2004/008491
(87) International publication number: WO 2004/110405

(57) **Abstract**

This invention provides a drug nanoparticle 7 obtained by irradiating laser beam 3 to a solid target 2 composed of drug powder so as to release the drug as downsized component particle from the solid target 2, wherein the drug nanoparticles 7 has an average diameter of 100 nm or less. According to the above structure, there can be provided: a drug nanoparticle having a high bioavailability, a high purity and an excellent handling property in a case where the drug nanoparticle is used as medical agent, agricultural chemical (agrichemical), chemical fertilizer or the like. The method of manufacturing the drug and the medical agent manufacturing apparatus are capable of effectively manufacturing the drug nanoparticles through simple manufacturing steps.

## Description

### Technical Field

The present invention relates to a drug nanoparticle, a method of manufacturing a medical agent and a medical agent manufacturing apparatus, more particularly to a drug nanoparticle having high bioavailability (BA), high purity and an excellent handling property in a case where the drug nanoparticle is used as medical product, agricultural chemical (agrichemical), chemical fertilizer or the like. Further, the present invention relates to a method of manufacturing the medical agent and the medical agent manufacturing apparatus capable of effectively manufacturing the medicine through simple manufacturing steps.

### Background Art

In the field of medical product, agricultural chemical, chemical fertilizer or the like, there have been conventionally few cases where a drug is used as it is in a form of the raw material. Almost all of the drug is subjected to a drug formulation by being controlled to have a specified particle size, or by being combined with other additives in accordance with an intended purpose, and then the formulated drug is administrated or dosed to a living body or the like at the first time.

Particularly in the field of drug formulation, there has been raised a great demand to increase the bioavailability of the drug in the living body thereby to intensify a therapeutic effect, and a demand to suppress an adverse effect of the drug by reducing an amount of drug to be administrated into the living body.

Further, in the field of medical product, there has been widely adopted a drug formulation in which the drug powder is combined with other excipient or carrier substrate without impairing any pharmacological active function for the purpose of controlling the physical-chemical properties such as stability, solubility, dissolution rate, wettability, dispersibility, flowability, packing property, compressive formability or the like of the drug.

Furthermore, in these drug formulation fields, there has been adopted a method in which a mechanical impact type finely pulverizing equipment or a milling equipment for applying a large impact-compressive force or a shear stress to the drug is used, and the drug is finely pulverized for a long time of period so as to reduce a particle (grain) size thereof or to lower a crystalline property of the particle structure for the purpose of improving the bioavailability of the drug.

However, even if the conventional mechanical impact type finely pulverizing equipment or the milling equipment is operated for a very long time, there has been a limitation of the particle size of the drug powder. Namely, an average particle size of the obtained drug powder is about 0.3 µm (300 nm) at the smallest. Therefore, there has been raised a problem such that the bioavailability at a diseased portion in the living body, to which a particularly fine drug powder is required to be administrated, is still in a lower level. For example, since an orally-taken medicine as asthma drug cannot be directly dosed into a lung and is necessary to dose in a form of a tablet to supply the drug through blood, there is an disadvantage such that an absorption rate of the drug is very small and a dosing amount of the drug is greatly increased, thus being liable to cause seriously adverse effects.

Further, even in a case where the drug powder (drug particle) is made to increase the dissolution rate thereof by reducing the physical size (dimension) of the drug, the aforementioned problems would not be solved. In order to drastically improve the bioavailability of the drug, a permeability (penetrating property) of the drug at cell membrane becomes an important factor in addition to an increase of the dissolution rate of the drug. In order to improve the permeability of the drug at the cell membrane, it is required not only to simply reduce the physical size of the drug, but also required a chemical interaction between the drug and a surface of the cell membrane. However, in case of a simple substance of the drug, there has been also posed a problem such that the chemical interaction between the drug and the surface of the cell membrane cannot have been expected. Namely, the chemical interaction was too small to lead directly to drastic improvement of the bioavailability of the drug.

Further, conventionally, the drug is pulverized for a long time by utilizing a mechanical impact type finely pulverizing equipment or milling equipment which applies a large impact-compressive force or a shearing stress to the drug. Therefore, heat energy to be caused during the finely pulverizing operation becomes excessively large, so that the drug is thermally decomposed, and change into decomposed species. As a result, a purity of the drug is disadvantageously lowered, so that a pharmacological action (medical property) of the drug is also lowered. In addition, in some cases, there may be a fear that bad influence such as adverse effect or the like due to the decomposed species generated as by-product is increased. Particularly, in a case where the drug is an organic compound that is liable to be thermally decomposed, a long time pulverizing operation is impossible; and there has been posed a problem that an organic drug cannot be easily obtained.

Furthermore, a contamination (impurity contamination) caused from a pulverizing vessel used during the long time pulverizing/milling treatment becomes also a serious technical problem to be solved so as not to lower the purity of the drug. Still further, when the drug is finely pulverized to form nano-sized particles having an average diameter of 100 nm or less, the pulverized particles are liable to scatter and the packing property of the particles is lowered. The pulverized particles are liable to agglomerate to each other by attracting ambient molecules, so that there may be posed a new problem such that a handling property of the drug is disadvantageously lowered.

The present invention had been achieved for solving the aforementioned conventional problems, and an object of the present invention is to provide a drug nanoparticle, a method of manufacturing a medical agent and a medical agent manufacturing apparatus, more particularly provide to a drug nanoparticle having high bioavailability, high purity and an excellent handling property in a case where the drug nanoparticle is used as medical product, agricultural chemical (agrichemical), chemical fertilizer or the like. Further, another object of the present invention is to provide a method of manufacturing the medical agent and the medical agent manufacturing apparatus capable of manufacturing the drug through simple manufacturing steps with a high production efficiency.

### Disclosure of Invention

In order to achieve the above object, the present inventors have studied various finely pulverizing methods, and comparatively reviewed the influences of the respective pulverizing methods and operating conditions thereof on the powder characteristics. As a result, the inventors have obtained the following findings. That is, when adopting a pulsed laser deposition method comprising the steps of: irradiating an ultraviolet pulsed laser beam onto a solidified target composed of the drug; and breaking intermolecular bonds between the components of the drug, it becomes possible to generate ultrafine drug particles having an average particle size of 10 - 100 nm while suppressing a temperature rise of the drug, even if the particles are composed of organic drug which is liable to be thermally decomposed easily. Hence, there can be efficiently obtained drug nanoparticles having a drastically improved bioavailability.

Further, the following findings were also obtained. Namely, when thus obtained drug nanoparticles are directly and continuously deposited in situ to surfaces of the other excipient particles (carrier particles), a composite nanoparticle can be efficiently manufactured through one step treatment. In addition, it becomes possible to stably handle the drug nanoparticles.

Furthermore, when the laser beam is irradiated to a solid target (pressed compact) composed of drug and protein thereby to prepare a drug-protein composite particle, the permeability of the drug at cell membrane is greatly improved due to the chemical interaction to the cell membrane through the protein, thereby to drastically increase the bioavailability of the drug. The present invention has been accomplished based on the above findings.

That is, the present invention provides a drug nanoparticle obtained by irradiating laser beam to a solid target (pressed compact or molded body) composed of drug powder so as to release the drug as nanoparticles from the solid target (pressed compact), wherein the drug nanoparticle has an average diameter of 100 nm or less.

A drug-protein nanocomposite according to the present invention is a drug-protein nanocomposite obtained by irradiating laser beam to a solid target composed of a mixture of drug powder and protein so as to release the drug and the protein as nanoparticles from the pressed compact, wherein each of the drug nanoparticles and the protein nanoparticles have an average diameter of 100 nm or less.

In the present invention, PLD (Pulsed Laser Deposition) method is used as a means for finely pulverizing the drug, protein or the like, nanoparticles having an average diameter of 100 nm or less, preferably 30 nm or less, which is unobtainable by any conventional pulverizing equipments, can be realized. Therefore, the bioavailability of the drug can be drastically improved.

Further, according to the PLD method, the temperature rise during the pulverizing treatment can be suppressed. Therefore, even in a case where the drug is composed of an organic compound, the thermal denaturization or decomposition hardly occur, and the drug nanopartides having a high product purity can be obtained.

Further, in the medical product field, when the drug is made to be nano size, the drug can be selectively dosed or administrated to only a diseased portion. As a result, there can be expected novel improving effects in drug delivery such as improved effect of the drug, suppression of the adverse effect or the like.

For example, it is well known that there is a difference between normal cell and cancer cell in a living body with respect to a penetration capacity and permeability of the drug at cell membrane. Such properties are different and varied in accordance with the particle diameter of the drug. Therefore, when a drug having a fine particle diameter is penetrated to only cancer cell, such drug can be intensively supplied to only the cancer cell, so that it becomes possible to drastically improve the bioavailability. In addition, an amount of the drug to be dosed into a living body can be greatly decreased, so that the adverse effect of the drug can be effectively suppressed.

In the above drug nanoparticle, it is preferable that the solid target (pressed compact) contains protein in addition to the drug powder. In this case, a drug-protein composite particle can be formed by irradiating laser beam to the solid target composed of drug and protein. When the particle is composed of only drug, there are cases where a chemical interaction between the drug and the surface of the cell membrane cannot be expected. However, according to this drug-protein composite particle, the chemical interaction can be expected through the action of the protein. Therefore, the permeability of the drug at the cell membrane can be greatly improved, so that the bioavailability of the drug can be drastically intensified.

In this connection, a presence or absence of above the chemical interaction between the drug and the protein can be confirmed by the following method. Namely, with respect to each of the solid target before the pulsed laser deposition and the drug-protein composite particle generated after the pulsed laser deposition, a chemical analysis is conducted by utilizing Fourier transform infrared absorption spectroscopy, thereby to obtain spectra peaks each indicating an absorbance of infrared ray with respect to respective wave numbers. As a result, if a change or difference in the spectra peaks between before and after the deposition is small, it can be confirmed and determined that there is no chemical interaction between the drug and the protein.

An average diameter of the drug nanoparticles according to the present invention is measured in accordance with the following method. That is, a micro-grid is prepared by adhering a collodion film onto a copper mesh. Then, particles are deposited onto the micro-grid by PLD method. With respect to a photograph of the deposited particles taken by a transmission electron microscope (TEM), an image analysis is conducted. With respect to 10 particles arbitrary selected from the particle images, diameters of circles each circumscribing the respective particle images are measured, and an average value of the measured diameters of the circles is defined as the average diameter of the drug nanoparticles.

In the above drug nanoparticle of the present invention, a part of the drug may be decomposed and changed to decomposed species. However, it is preferable that a ratio of the decomposed drug with respect to a total amount of the drug shall be as small as possible.

When the above ratio of the decomposed drug becomes large, a purity of medical product becomes low and there may be a case where harmful effects such as adverse reaction or the like caused by the decomposed species becomes remarkable, thus being not preferable.

Originally, as described hereinbefore, according to the PLD method, the temperature rise during the pulverizing treatment can be suppressed in comparison with the conventional mechanical impact type pulverizing method. Therefore, even in a case where the drug is composed of an organic compoud, the thermal decomposition hardly occurs, and the drug nanoparticles having a high purity can be obtained.

Whether or not the drug nanoparticle is decomposed can be judged in accordance with the following method. That is, for example, the drug is dissolved in acetone thereby to prepare one sample, while drug thin film deposited on a glass substrate by PLD method is dissolved thereby to prepare another sample. With respect to each of the samples, nuclear magnetic resonance (NMR) spectrum is obtained respectively. Then, both of the spectra obtained before and after the PLD treatment are compared. At this time, when a new peak corresponding to the decomposed species generated by the decomposition of the drug is observed in the spectrum obtained after the PLD treatment, it is judged that the drug nanoparticle is changed into the decomposed species. On the contrary, when the peak is not observed, it is judged that the decomposition did not take place.

Further, whether or not the drug nanoparticle is decomposed and an amount of the decomposed species can be also analyzed in accordance with the following high performance liquid chromatography (HPLC). Namely, the solid target (pressed compact) before the pulsed laser deposition (PLD) is dissolved into a solvent as a mobile phase thereby to prepare one solution, while the drug-protein composite particle generated after the deposition is dissolved into the solvent thereby to prepare another solution. Then, each of the solutions is flown into a column packed with separating medium, respectively. When the retention time of the drug is the same before and after PLD, with no new peaks, it can be judged that decomposed species is not generated, or is under the detection limit of HPLC.

In the present invention, "drug" denotes a substance having a specified pharmacological effect, while "medical agent" denotes a composite product prepared by combining the drug with an excipient or the like.

The present invention provides a method of manufacturing a medical agent, comprising the steps of: irradiating a laser beam to a solid target composed of drug components under an inert gas atmosphere of reduced pressure, and breaking intermolecular bonds of the drug components thereby to release the drug as molecules and clusters; generating nanoparticles having an average diameter of 100 nm or less from these molecules and clusters; and adhering and depositing the nanoparticles onto a surface of excipient particles.

Furthermore, in another aspect of the present invention, there is provided a medical agent manufacturing method in which the solid target (pressed compact) contains protein in addition to a drug powder, the laser beam is irradiated to the solid target (pressed compact) composed of drug and protein, and drug-protein composite nanoparticles are adhered to surfaces of the excipient particles thereby to form a medical agent. Concretely, the manufacturing method comprises the steps of: irradiating the laser beam to the solid target composed of solid drug components and protein under a reduced pressure of inert gas atmosphere, and breaking intermolecular bonds between the drug components and the protein thereby to release the drug and the protein as molecules and clusters; generating nanoparticles of the drug and the protein each having an average diameter of 100 nm or less from the molecules and clusters; and adhering the nanoparticles, as a composite of drug particle and protein particle, onto a surface of the excipient particles.

In the above step of releasing the drug component and the protein as molecules and clusters and the step of generating the nanoparticles of the drug and the protein, it is preferable to execute the steps under an inert gas atmosphere with a reduced pressure of about 1 - 1000 Pa for the purpose of suitably control the average diameter (average grain size) of the nanoparticles. As the inert gas atmosphere, nitrogen gas, argon gas, xenon gas, helium gas or the like are used.

As the above excipient particle, as far as the excipient is harmless and inactive (having no pharmacological activity) against a living body and is chemically and physically stable, the excipient is not particularly limited. For example, the excipient such as lactose, glucose, dextrin, cornstarch, potato starch or the like are preferably used.

Particularly, when the protein is used as the above excipient and a drug-protein composite particles are formed by irradiating the laser beam onto the solid target composed of drug and protein, subsequently by releasing and combining the fine protein particles with the fine drug particles, the permeability of the drug at the cell membrane can be greatly improved. As a result, the bioavailability of the drug can be drastically increased.

Further, according to the above medical agent manufacturing method, since the prepared drug nanoparticle or the drug-protein composite nanoparticle are directly coated onto the excipient thereby to produce a composite medical agent, a scattering or a re-agglomeration of the nanoparticles can be effectively suppressed. As a result, a problem regarding handling property of the nanoparticles can be effectively solved.

Furthermore, in a case where the solid target (pressed compact) is prepared by pressing the drug powder in the above medical agent manufacturing method, the solid target can be also prepared by a die-molding method in which only a pressing force is applied to the drug powder packed in a die thereby to prepare a solid target. However, there are some cases where a sufficient structural strength of the solid target cannot be obtained for some particular drug powders even if the drug powders are subjected to only a pressing operation. In this connection, there has been often arose a case where a cracking, so-called capping, is liable to occur when the solid target (pressed compact) is taken out from the die.

In order to prevent such cracking, it is effective to prepare the solid target (pressed compact) by utilizing a hot pressing method in which the drug powder is pressed and simultaneously heated to a temperature lower than a melting point of the drug powder. As an alternative method, it is also effective to utilizing a melt quench method in which the drug powder is pressed and simultaneously heated to a temperature immediately below the melting point of the drug powder, so that the drug powder is partially molten, thereafter the molten drug is rapidly quenched and solidified thereby to prepare a solid target as the pressed compact.

According to the method of manufacturing the medical agent, the nanoparticles of the drug and the protein are released from the target, the drug nanoparticles or the drug-protein composite nanoparticles generated from the molecules and clusters directly adhered and deposited on the surface of the excipient. Therefore, a medical agent as a composite composed of the nanoparticles or the composite nanoparticles and the excipient particles can be effectively manufactured through one process step.

In addition, the present invention provides a medical agent manufacturing apparatus comprising: a target comprising a pressed compact composed of drug powder; a laser generating equipment (laser oscillating device) for irradiating laser beam to the target so that intermolecular bonds of drug components are broken and the drug components are released from the target; a drug container for generating nanoparticles, having an average diameter of 100 nm or less, from the released drug components and for adhering the nanoparticles onto a surface of an excipient particles; and a vacuum chamber for accommodating the target and the drug container.

As a laser beam to be irradiated to the target, it is preferable to use an ultraviolet pulsed laser beam having a high energy density. As a condition of the laser irradiation, when a wavelength of the laser beam becomes short, irradiation energy of the laser beam becomes large and it is possible to increase the rate of the nanoparticles to be released per time. However, the wavelength within a range of 266 nm to 1064 nm is preferable. Further, when a laser output power is increased, it is possible to release the nanoparticles with higher efficiently. However, when the laser output power is excessively increased, the decomposed species are liable to be generated, and there may be a possibility of lowering a purity of the drug. In this regard, it is confirmed as a technical knowledge that the decomposed species are not generated if the laser output power is set within a range of 5 - 20 J/cm².

When the above ultraviolet pulsed laser beam is irradiated to a solid target, the intermolecular bonds between the drug component and the protein constituting the target are broken, so that the drug component and the protein are downsized to be fine drug particles each having a size of molecule or cluster, and then the fine drug particles are photo-chemically excited and released. Until a time when the released fine drug particles or the like reach the excipient particles or a substrate disposed so as to oppose to the target, the released fine drug particles or the like collide with molecules of the ambient gas and other downsized drug particles within the vacuum chamber. Then, the released fine drug particles or the like collide with surfaces of the excipient particles or the substrate, or the released fine particles or the like are agglomerated to each other. As a result, nanoparticles having an average diameter of 100 nm or less are deposited on the surfaces of the excipient particles or the substrate. Accordingly, there can be prepared a composite medical agent in which the drug nanoparticles are combined with the excipient particles.

As described hereinbefore, in order to control the average diameter of the drug nanoparticles to be 100 nm or less, it is preferable to set the ambient pressure in the vacuum chamber (vacuum container) to an inert gas atmosphere with a reduced pressure of about 1 to 1000 Pa. As the ambient gas, nitrogen gas or helium gas is particularly preferable.

There is a tendency that the diameter of the drug nanoparticle is increased in accordance with the increase of the above ambient pressure. Therefore, by appropriately controlling the above ambient pressure, the average diameter (grain size) of the drug nanoparticles to be generated by PLD method can be accurately controlled.

According to the above medical agent manufacturing apparatus, since the PLD treatment is performed within the vacuum vessel, the problem of contamination (impurity contamination) can be eliminated in comparison with the conventional mechanical pulverizing equipment using a conventional pulverizing vessel.

Particularly, the PLD method adopts a system in which an ultraviolet or infrared laser beam is irradiated to a solid target composed of drug or a pressed compact composed of the drug and protein, and the intermolecular bonds between the drug components and the protein are broken whereby the nanoparticles excited photo-chemically are released. Therefore, a temperature rise is suppressed, so that it is possible to downsize the drug components liable to be easily decomposed. As a result, a drug nanoparticle composed of an organic compound and having a stable quality can be easily manufactured.

Further, there is a case where the medical agent particles as composite material are manufactured by adhering the drug nanoparticles or the drug-protein composite nanoparticles onto an entire surface of the excipient particles. In this case, even if the PLD operation is performed in a state where the excipient particles are left at rest so as to oppose to the target, there may be a case where the drug nanoparticles or the drug-protein composite nanoparticles are mainly adhered to only one side surface (i.e. a surface directing to the target) of the excipient particles, so that it is difficult to uniformly form a depositing film onto the entire surface of the excipient particles.

To cope with this situation, for example, in a case where the drug nanoparticles or the drug-protein composite nanoparticles are deposited onto the surface of the excipient composed of materials such as granular lactose, glucose, dextrin, comstarch or the like thereby to form a dense film-like thin layer and to manufacture the composite particles, it is more preferable that the medical agent manufacturing apparatus further comprises a vibrating device for continuously or intermittently applying vibrations to the excipient particles or the like thereby to fluidize the excipient particles or the like.

When the vibrations are continuously or intermittently applied to the excipient particles (base material particles) or the like by the vibrating device, the base material particles are fluidized and a surface direction of each of the excipient particles is changed during the vibration, so that the thin film layer composed of the drug nanoparticles can be uniformly adhered and formed onto the entire surfaces of the respective excipient particles.

According to the above medical agent manufacturing apparatus, since the PLD treatment is performed within the vacuum vessel, the problem of the impurity contamination can be solved in comparison with the conventional mechanical pulverizing equipment using a conventional pulverizing vessel.

Further, in the conventional combining treatment for the downsized particles and the excipient particles, a step for downsizing the particle and a step for combining the particles with the excipient particles are separated and equipments for the respective steps are independently installed. Therefore, there have been arose the problems such that the contamination is liable to occur on the way of transferring the raw material or product between the respective operation steps, and the manufacturing steps are complicated.

In contrast to this, according to the medical agent manufacturing apparatus of the present invention, the laser beam is irradiated to the target composed of a predetermined drug component or the target composed of the drug and protein thereby to downsize the drug component or the like in the vacuum vessel, subsequently the nanoparticles generated from the drug component are directly deposited onto the surface of the excipient particles. Therefore, the medical agent as a composite having the thin layer (composed of fine nanoparticles) formed on the surface of the excipient particles can be remarkably easily manufactured through only one step consisting of PLD operation.

Furthermore, in the above medical agent manufacturing apparatus, the pressed compact composed of the drug and protein is used as the target, then the laser beam is irradiated to this pressed compact, and the drug-protein composite nanoparticles are adhered onto the surface of the excipient particles. In this case, the chemical interaction between the drug and a surface of cell membrane can be intensified through the action of the protein. Therefore, the permeability of the drug at the cell membrane can be greatly improved, so that the bioavailability of the drug can be drastically increased.

According to the drug nanoparticle, the medical agent manufacturing method or apparatus using the drug nanoparticle of the present invention, the invention adopts a method in accordance with PLD method comprising the steps of: irradiating ultraviolet laser beam to a solid target as the pressed compact composed of the drug; breaking the intermolecular bonds of the drug components constituting the target; releasing the downsized drug components excited photochemically; and generating the nanoparticles from the downsized drug components. Therefore, drug nanoparticles having a drastically improved bioavailability can be prepared. In addition, according to the PLD method, a temperature rise of the material is suppressed even if the laser beam is irradiated to the material, so that it is possible to downsize an organic compound that is liable to be thermally denatured or decomposed. As a result, a drug nanoparticle composed of organic compound and having a stable quality can be also easily manufactured.

### Brief Description of the Drawings

FIG. 1 is a cross sectional view showing a structure of one embodiment of the manufacturing method and the manufacturing apparatus using the drug nanoparticle according to the present invention.
FIG. 2 (A) and (B) are transmission electron microscope (TEM) photographs each showing a structure of a drug (PT: phenytoin) nanoparticles prepared in Example 1, and FIG. 2 (A) and (B) are also structural views of which an observation spot or an observation magnification is changed.
FIG. 3 (A) and (B) are TEM photographs each showing a structure of a drug (IM: indomethacine) nanoparticles prepared in Example 2, and FIG. 3 (A) and (B) are also structural views of which an observation spot or an observation magnification is changed.
FIG. 4 (A) and (B) are graphs showing nuclear magnetic resonance (NMR) spectra of a drug (PT: phenytoin) nanoparticles prepared in Example 1 before and after the PLD treatment, respectively.
FIG. 5 (A) and (B) are graphs showing NMR spectra of a drug (IM) nanoparticles prepared in Example 2 before and after the PLD treatment, respectively.
FIG. 6 (A) and (B) are graphs showing NMR spectra of a drug (EZ: ethenzamide) nanoparticles prepared in Example 3 before and after the PLD treatment, respectively.
FIG. 7 is a TEM photograph showing a structure of composite drug (PT) nanoparticles in which a film-like thin layer composed of dense drug (PT) nanoparticles is adhered onto a surface of a lactose particle as an excipient particle.
FIG. 8 (A) and (B) are TEM photographs each showing a structure of a drug (IM) nanoparticles prepared in Example 5, and FIG. 8 (A) and (B) are also structural views of which an observation spot or an observation magnification is changed.
FIG. 9 is a graph showing infrared absorption spectra of the drug (IM) composite nanoparticles prepared in Example 5 before and after the PLD treatment, respectively.

### Best Mode for Carrying out The Invention

Embodiments of the drug nanoparticles, the medical agent manufacturing method or apparatus using the drug nanoparticles of the present invention will now be concretely described with reference to the following Examples.

### [Starting Material]

At first, drug materials (starting materials) to be used in the respective examples were prepared as follows.

That is, the following four kinds of drug powders were prepared as starting materials for Examples 1 to 4 and experiments were conducted. The drug powders were:
① phenytoin powder (manufactured by Wako Junyaku Industry Co. Ltd.; and hereinafter referred simply as "PT") as anti-epilepsy agent;
② γ -indomethacine powder (manufactured by Sigma Chemical; and hereinafter referred simply as "IM") as anti-pyreticanalgesics agent and anti-inflammatory agent;
③ ethenzamide powder (manufactured by Aldrich; and hereinafter referred simply as "EZ") as anti-pyreticanalgesics agent; and
④ ibuprofen powder (manufactured by Tokyo Kasei Industry Co. Ltd.; and hereinafter referred simply as "IB") as anti-pyreticanalgesics agent and anti-inflammatory agent.

### [Preparation of Target for Pulsed Laser Deposition (PLD)]

2 grams of each of the four drug powders was packed in a molding die having an inner diameter of 30 mm. Next, the packed powder was pressed at a pressing force of 55.5 MPa for 3 minutes using a hydraulic pressing machine (manufactured by Mori Testing Machinery) thereby to form molded bodies, respectively.

In this regard, in cases of the PT pressed compact for Example 1 and IM pressed compact for Example 2 that were prepared by applying only the pressing force, when the molded bodies were taken out from the molding die, a whole area crack (full crack), so-called, "capping" occurred. Therefore, it was confirmed that strength of each of the molded bodies for Examples 1 and 2 was insufficient.

To cope with this situation so as to improve the molding property, the inventors adopted a hot pressing method in which a pressing force was applied to the packed powder and simultaneously the packed powder was heated to a temperature below the melting point of the drug, thereby to prepare a solid target having an increased structural strength. The pressing condition and the melting points of the drug powders are shown in Table 1 hereunder.

**[Table 1]**

| Sample No. | Name of Drug Name of Drug | Hot Pressing Condition | | Melting Point (°C) |
|---|---|---|---|---|
| | | Pressing Force (MPa) | Heating Temperature (°C) | |
| Example 1 | PT (phenytoin) | 55.5 | 200 | 298 |
| Example 2 | IM (γ-indomethacin) | 55.5 | 80 | 155,162 |
| Example 3 | EZ (ethenzamide) | 55.5 | - | 132 |
| Example 4 | IB (ibuprofen) | 55.5 | - | 75 |

As is clear from the results shown in above Table 1, in case of the drug powder having a relatively high melting point and a poor densification property, when adopting the hot pressing method in which the drug powder was heated to a temperature below the melting point of the drug and simultaneously the pressing force was applied to the drug powder, it was possible to prepare a solid target having a sufficient density and a high structural strength.

### [Pulsed Laser Deposition (PLD)]

### ① Generation of Drug Nanoparticles

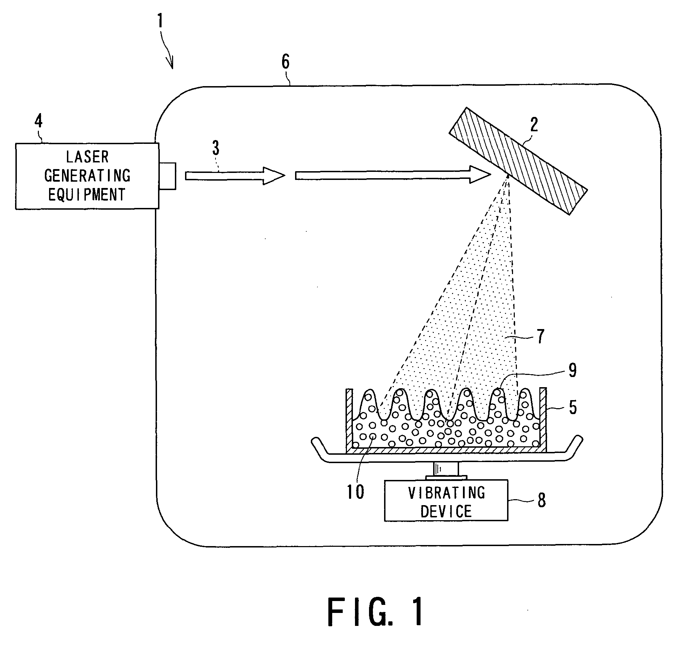

Each of thus prepared targets for the respective Examples was loaded within a medical agent manufacturing apparatus 1 shown in FIG. 1 and then PLD operation was conducted for each target, whereby drug nanoparticles were deposited to a micro grid. The above micro grid was prepared by adhering a collodion film to a copper mesh member.

As shown in FIG. 1, the medical agent manufacturing apparatus (pulsed laser deposition system) 1 according to Examples comprises: a target 2 comprising a pressed compact composed of drug powder; a laser generating equipment 4 for irradiating laser beam 3 to the target 2 so that intermolecular bonds of drug components are broken and the drug components are released from the target 2; a drug container 5 for generating drug nanoparticles 7, having an average diameter of 100 nm or less, from the released drug components and for depositing the drug nano particles 7 onto a surface of excipient particles 10; and a vacuum chamber 6 for accommodating the target 2 and the drug container 5. In addition, this medical agent manufacturing apparatus 1 further comprises a vibrating device (vibration generator) 8 for applying vibration to the excipient particles 10 to which the drug nanoparticles 7 are adhered, so that the drug nanoparticles 7 deposited to the excipient particles 10 are fluidized.

The PLD operation was conducted by utilizing the laser generating equipment (H-114005) 4 provided to the above medical agent manufacturing apparatus 1. As a laser beam (laser light) to be irradiated, a fourth harmonic wave (wavelength: 266 nm, pulse frequency: 10 Hz, pulse width: 6-8 ns) of Nd: YAG laser was used. A laser output power was changed within a range of 5 - 20 J/cm². An irradiation time (treating time) of the laser beam was set to 1 hour thereby to conduct the experiment for the respective targets.

Further, the ambient gas charged into the vacuum chamber (vacuum container) 6 was nitrogen gas, and the pressure of the ambient gas was changed from 1 Pa to 1000 Pa.

A state of the drug nanoparticles deposited to the micro grid was observed by means of a transmission electron microscope (TEM: TECNAI F20 manufactured by PHILLIPS CORP.), and an average diameter of each of thus generated drug nanoparticles was measured. As a result, the average diameter of the respective drug nanoparticles of Examples 1 - 4 were within a range of 15 to 20 nm.

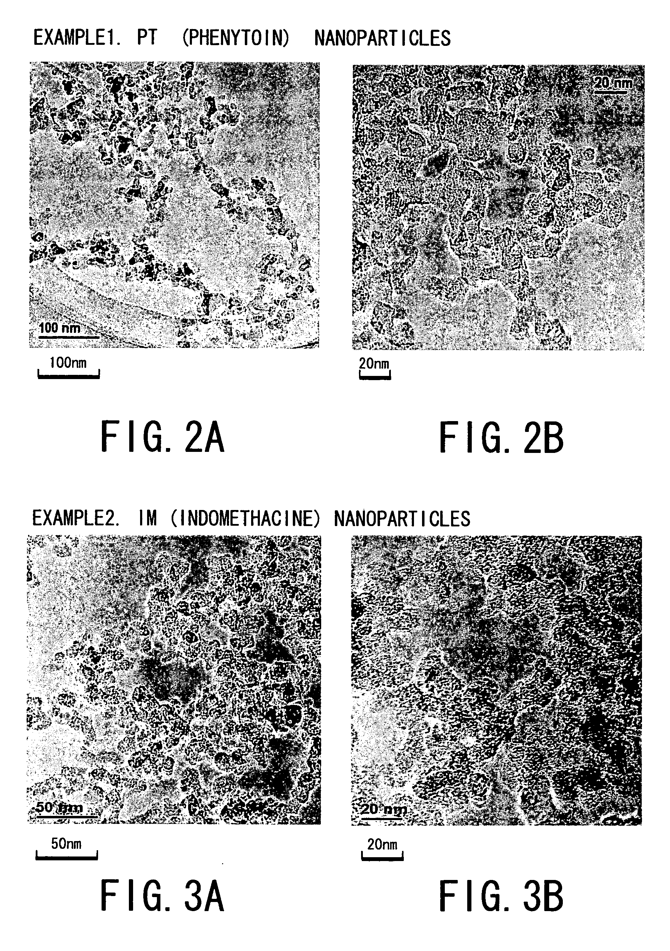

FIG. 2 (A) and (B) are TEM photographs each showing a structure of a drug (PT) nano particles prepared in Example 1, and FIG. 2 (A) and (B) are also structural views of which an observation spot or an observation magnification is changed. As shown in FIG. 2 (A) and (B), according to Example 1, extremely fine PT nanoparticles having an average diameter of 15 nm and adhered in a chain-agglomerated form to the surface of the micro grid were obtained. Therefore, it was confirmed that the drug nanoparticles could be effectively manufactured.

FIG. 3 (A) and (B) are TEM photographs each showing a structure of a drug (IM) nano particles prepared in Example 2, and FIG. 3 (A) and (B) are also structural views of which an observation spot or an observation magnification is changed. As shown in FIG. 3 (A) and (B), according to Example 2, extremely fine IM nanoparticles having an average diameter of 18 nm and adhered in a chain-agglomerated form to the surface of the micro grid were obtained. Therefore, it was confirmed that the drug nanoparticles could be effectively manufactured.

Furthermore, in order to investigate whether thermal denaturalization or decomposition of the drug had occurred or not, the following spectroscopic analysis was conducted with respect to each of the drug nanoparticles prepared through PLD treatment in Examples 1 to 4. Namely, each of the drug nanoparticles deposited on a glass substrate was dissolved in acetone (Acetone-d6; manufactured by Aldrich) thereby to prepare the respective sample solutions. Then, each of the sample solution was analyzed by means of a nuclear magnetic resonance spectroscopic analyzer (¹H-NMR: Varian NMR Spectrometer System; 300 MHz). Another NMR spectroscopic analysis was conducted with respect to a drug powder in a stage of a starting material before PLD treatment as in the same manner as described above. On the basis of the above comparative analyses, whether or not a peculiarly new peak to be occurred due to the decomposition or the thermal denaturalization of the drug material is formed in the spectrum after the PLD treatment.

As a result, regarding the respective drug nanoparticles of Examples 1 to 4, the peculiarly new peak to be occurred due to the decomposition or the thermal denaturalization of the material was not observed at all in any spectra after the PLD treatments.

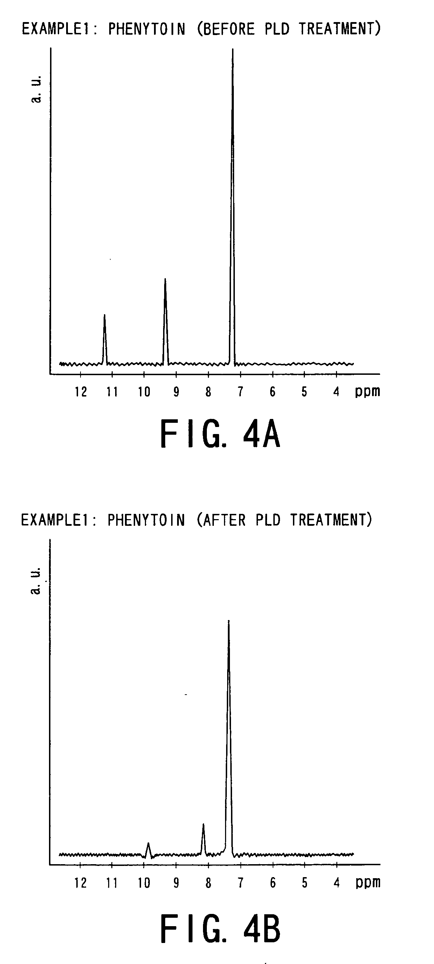

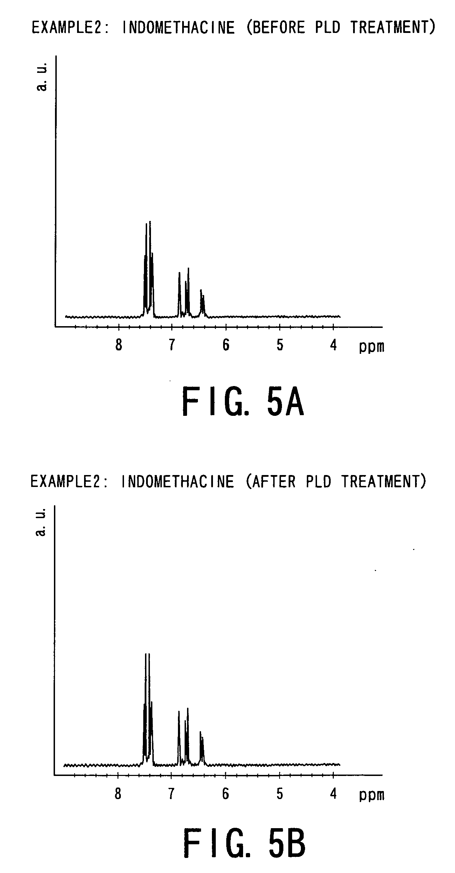

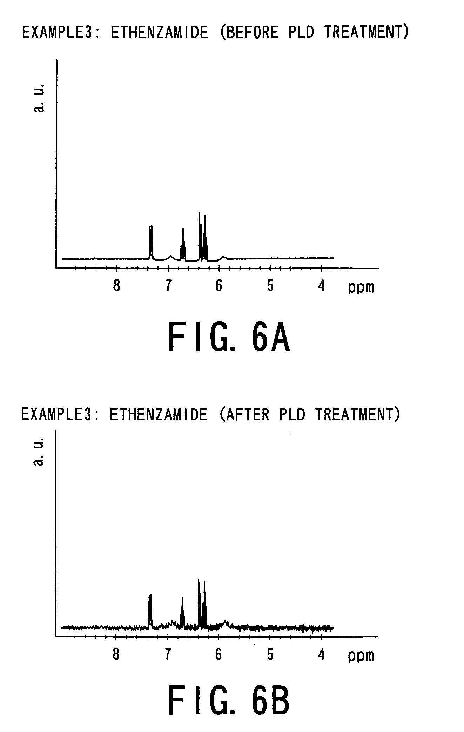

FIG. 4 (A), (B) to FIG. 6 (A), (B) are graphs showing NMR spectra of the drug nanoparticles prepared in Examples 1 to 3 at a time before and after the PLD treatment, respectively. In case each of the IM nanoparticles of Example 2 shown in FIG. 5 (A) and (B) and the EZ nanoparticles of Example 3 shown in FIG. 6 (A) and (B), the NMR spectrum at a time before the PLD treatment is exactly the same as that of after the PLD treatment.

On the other hand, in case of the PT nanoparticles of Example 1 shown in FIG. 4 (A) and (B), a position of the peak is somewhat deviated to each other. However, there are no peculiar peaks resulting from the decomposed species. Therefore, when taking a degree of accuracy of the NMR spectroscopic analyzer into consideration, even if the decomposed species were generated by the laser radiation, it was confirmed that each amount of the respective decomposed species was less than a specified detecting limit of the NMR spectroscopic analyzer.

### ② Preparation of Composite Particles

Each of the targets prepared for the respective Examples was loaded within the medical agent manufacturing apparatus shown in FIG. 1, and then PLD operation was performed whereby the drug nanoparticles were directly deposited onto the surfaces of excipient particles.

More concretely, a PT target was used as a target 2 composed of drug (PT phenytoin), while lactose powder having an average diameter of 5 µ m was used as the excipient particles. As a laser beam to be irradiated, a fourth harmonic wave of Nd : YAG laser was used. A laser output power was set to 10 J/cm², and an irradiation time (treating time) of the laser beam was set to 1 hour thereby to conduct the experiment for the target 2.

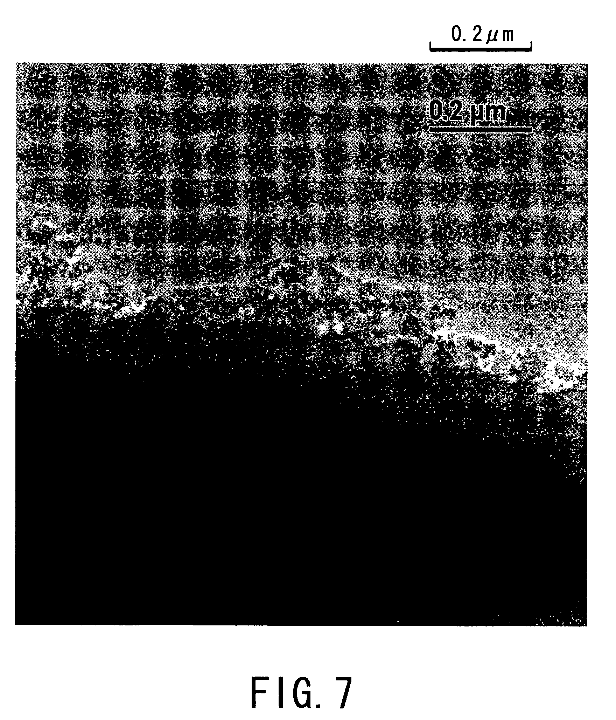

During the experiment, vibrations were intermittently applied to the excipient particles 10 in accordance with a time schedule in which the vibrating device 8 was operated for 10 seconds after the laser beam irradiation is continued for 1 minute and 50 seconds, whereby the dilution agent particles 10 fluidized. In addition, the ambient gas charged into the vacuum chamber 6 was nitrogen gas and a pressure of the ambient gas was set to 1000 Pa, thereby to conduct the PLD operation. As a result, as shown in FIG 7, there could be obtained a composite particle in which a film-like thin layer having a thickness of 0.1 - 0.4 *µ*m and composed of close-packed PT nanoparticles was deposited onto a surface of the lactose particle.

Next, a drug-protein composite nanoparticle of Example 5 will be explained. The composite particle was prepared by a method comprising the steps of: preparing a pressed compact as a solid target composed of drug and protein; irradiating laser beam onto the solid target, releasing the downsized particles composed of protein and the downsized particles composed of the drug; and combining the downsized protein particles with the downsized drug particles thereby to manufacture the drug-protein composite nanoparticles.

### [Starting Material]

As a drug material (starting material) used in Example 5, γ -indomethacin powder (hereinafter referred simply as "IM") used as drug material in Example 2 and bovine serum albumin powder (manufactured by Signa-Aldrich. and hereinafter referred simply as "BSA") as protein were prepared.

### [Preparation of Target for PLD operation]

The above IM powder as drug material and the BSA powder were blended at three blending ratios (weight ratio of drug and protein) of 1:9, 5:5 and 9:1 thereby to prepare three kinds of blended materials. Thereafter, each of the blended materials was mixed and pulverized for one hour by means of a vibration ball mill thereby to prepare three kinds of mixed materials.

2 grams of each of the three kinds of the mixed materials was packed in a molding die having an inner diameter of 25 mm. Next, the packed powder was pressed at a pressing force of 20 MPa and a temperature of 50°C for 5 minutes using a hydraulic pressing machine (manufactured by Mori Testing Machinery) thereby to form molded bodies, respectively.

### [Pulsed Laser Deposition (PLD)]

Each of thus prepared three kinds of the targets for Example 5 was loaded within a medical agent manufacturing apparatus 1 shown in FIG. 1 and then PLD operation was conducted for each target, whereby composite particles (BSA-IM composite particles) composed of drug particles and the protein particles were deposited to a micro grid. The above micro grid was prepared by adhering a collodion film to a copper mesh member.

The PLD operation was conducted by utilizing a laser generating equipment 4 provided to the above medical agent manufacturing apparatus 1. As the laser generating equipment 4, an Nd:YAG laser generating equipment (manufactured by New Wave Research Corp.) was used. As a laser beam to be irradiated, a laser beam having a fundamental wavelength: 1064 nm, pulse frequency: 10 Hz, pulse width: 5 ns) of Nd : YAG laser was used. A laser output power was set to 5 J/cm². An irradiation time (treating time) of the laser beam was set to 1 hour thereby to conduct the experiment for the respective targets. Further, the ambient gas charged into the vacuum chamber (vacuum container) 6 was helium (He) gas, and the pressure of the ambient gas was set to 100 Pa.

A state of the drug-protein composite nanoparticles of Example 5 adhered to the micro grid was observed by means of a transmission electron microscope (TEM: TECHNAI F20 manufactured by PHILLIPS CORP.), and an average diameter of each of thus generated drug nanoparticles was measured. As a result, each of the average diameter of the drug particles and the protein particles was within a range of 100 nm or less in the drug-protein composite nanoparticles of Example 5.

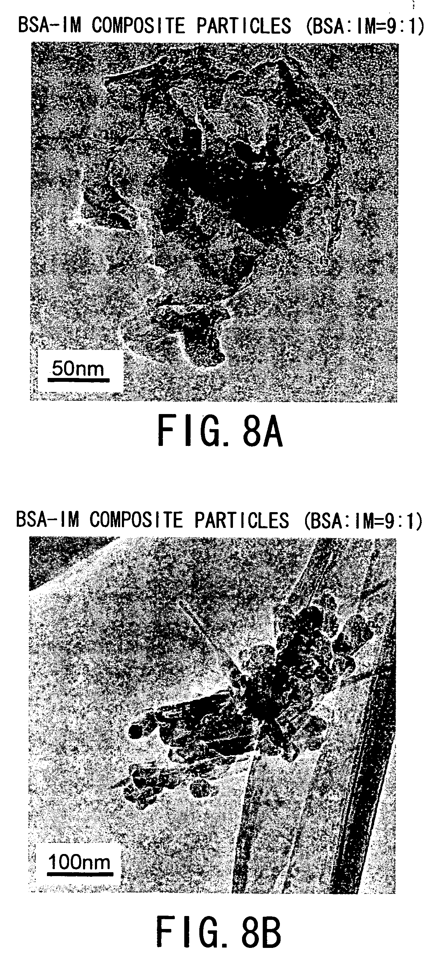

FIG. 8 (A) and (B) are TEM photographs each showing a structure of a BSA-IM composite particles of Example 5 in which the weight ratio of BSA: IM was set to 9:1, and FIG. 8 (A) and (B) are also structural views of which an observation spot or an observation magnification is changed. As shown in FIG. 8 (A) and (B), according to Example 5, extremely fine IM drug nanoparticles and BSA protein particles each having an average diameter of 100 nm or less and deposited in a chain-agglomerated form to the surface of the micro grid were obtained. Therefore, it was confirmed that the drug-protein composite nanoparticles could be effectively manufactured.

Further, in order to confirm whether or not an chemical interaction between the drug and the protein constituting thus prepared BSA-IM composite particles (medicine composite nanoparticles) was occurred, an infrared absorbance of the material mixtures before PLD operation and the composite particles after PLD operation was measured by utilizing a Fourier transform infrared absorption spectrometer (FT-IR analyzer, FTS-175 manufactured by BIO-RAD Corp.) through the following procedure. Transmission type KBr method was used. Background spectrum was measured by 50 mg of KBr, and sample spectra were measured by mixing 0.5 mg of KBr. The infrared absorbance was measured under the conditions of integrating scan number of 256 and at a resolution of 1 cm⁻¹.

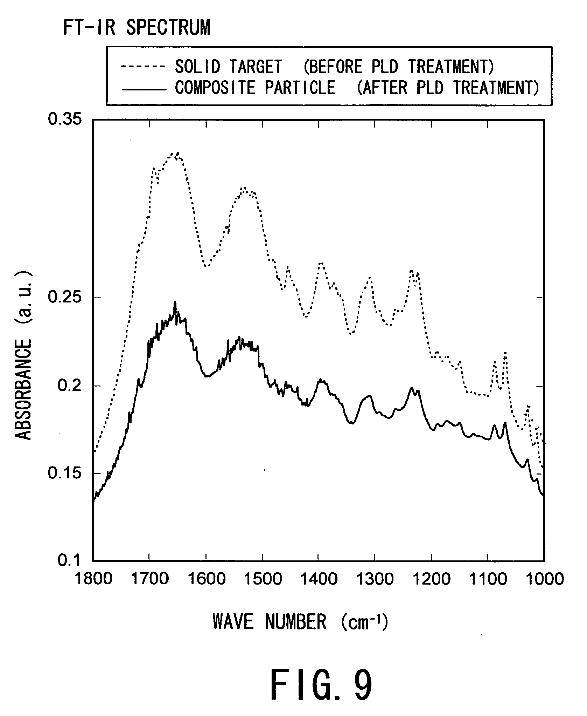

Fourier transform infrared absorption spectra of the material mixture (pressed compact) before PLD treatment and the composite particles after PLD treatment are shown in FIG 9.

As is clear from the results shown in FIG 9, the FT-IR spectrum after the PLD treatment has almost the same spectrum and absorbance peaks as those in FT-IR spectrum before the PLD treatment, and the interaction between the drug and the protein is not detected as a chemical shift. Therefore, it is considered that the interaction between the drug and the protein is only a physical interaction and the chemical interaction is not occurred at all.

In this connection, FIG. 9 shows infrared absorption spectra, before and after PLD operation, of the BSA-IM composite particles (drug composite nanoparticles) prepared by setting the weight ratio of BSA : IM to 9:1. However, even in a case where the weight ratio of BSA : IM was set to 1:9, almost the same spectra peaks were obtained. Therefore, it could be confirmed that the interaction between the drug and the protein was small regardless of a content of the drug.

Further, in order to investigate whether thermal denaturalization or decomposition of the drug had occurred or not, the following high performance liquid chromatographic (HPLC) analysis was conducted with respect to each of the drug nanoparticles prepared through PLD treatment in Examples 5, by utilizing a high performance liquid chromatographic analyzer (HPLC analyzer: HP-1500; manufactured by Hewlett Packard Corp.). Namely, methanol and a phosphoric acid solution having a concentration of 1 mMol were blended at a volumetric ratio of 7:3 thereby to prepare a solvent as a mobile phase. A flow rate of the solvent was set to 1 mL/min. As a column, L-column ODS having a size of 4.6 × 150 mm was used. An UV detecting wave length was set to 254 nm. Then, 2 mg of the drug composite nanoparticles was dissolved into 2 mL of the same solvent as the above solvent thereby to prepare a sample, and 1 *µ*L of the sample was injected into the column.

As the results of the analyses utilizing the high performance liquid chromatographic analyzer (HPLC analyzer), it was confirmed that the decomposed species were not generated at all or the amount thereof was less than the detecting limit of the analyzers. Therefore, according to the respective drug composite nanoparticles of Example 5, the medical agent comprising a high purity drug and having a less adverse effect can be effectively obtained.

As described above, according to the drug composite nanoparticles of Example 5, since the laser beam is irradiated to the pressed compact composed of a mixed powders of the drug and the protein and then the drug and the protein are released as the downsized components, the decomposed species due to thermal denaturalization or decomposition caused by the laser beam irradiation is not generated, so that the drug-protein composite nanoparticles (medical agent particles) having a high purity can be effectively obtained. In this drug-protein composite nanoparticles, the chemical interaction between the drug and the protein is not generated, and the drug and the protein are combined by only a physical interaction. Further, the decomposed species is not formed at all, and it becomes possible to remarkably improve the permeability of the drug at cell membrane.

### Industrial Applicability

As described above, according to the drug nanoparticle, the medical agent manufacturing method or apparatus using the drug nanoparticle of the present invention, the invention adopts a method in accordance with PLD method comprising the steps of: irradiating ultraviolet laser beam to a solid target as the pressed compact composed of the drug; breaking the intermolecular bonds of the drug components constituting the target; releasing the downsized drug components excited photochemically; and generating the nanoparticles from the downsized drug components. Therefore, nano-sized drug particles having a drastically improved bioavailability. In addition, according to the PLD method, a temperature rise of the material is suppressed even if the laser beam is irradiated to the material, so that it is possible to downsize an organic compound that is liable to be thermally decomposed. As a result, a drug nanoparticle composed of organic compounds and having a stable quality can be also easily manufactured.

## Claims

1. A drug nanoparticle obtained by irradiating laser beam to a solid target composed of drug powder so as to release the drug as nanoparticles from the solid target, wherein said drug nanoparticles have an average diameter of 100 nm or less.

2. The drug nanoparticles according to Claim 1, wherein said drug is composed of organic compound.

3. A drug-protein nanocomposite obtained by irradiating laser beam to a solid target composed of a mixture of drug powder and protein so as to release the drug and the protein as nanocomposites from the solid target, wherein each of drug nanoparticles and protein nanopartides constituting the nanocomposites have an average diameter of 100 nm or less.

4. The drug-protein nanocomposite according to Claim 3, wherein said drug is composed of an organic compound.

5. A method of manufacturing a medical agent, comprising the steps of:
irradiating a laser beam to a solid target composed of drug components under an inert gas atmosphere of reduced pressure, and breaking intermolecular bonds of said drug components thereby to release said drug as nanoparticles;
generating nanoparticles having an average diameter of 100 nm or less; and
depositing said nanoparticles onto a surface of excipient particles.

6. A method of manufacturing a medical agent, comprising the steps of:
irradiating a laser beam to a solid target composed of drug components and protein under a reduced pressure of inert gas atmosphere, and breaking intermolecular bonds between said drug components and said protein thereby to release said drug and said protein as nanocomposites:
generating nanoparticles of the drug and the protein each having an average diameter of 100 nm or less; and
depositing said nanoparticles as nanocomposites onto a surface of excipient particles.

7. The method of manufacturing a medical agent according to Claim 5 or 6, wherein said average diameter of the generated drug nanoparticles is controlled by adjusting species and pressure of said inert gas atmosphere.

8. The method of manufacturing a medical agent according to Claim 5, wherein said solid target is prepared by pressing the drug powder and simultaneously heating the drug powder to a temperature lower than a melting point of the drug powder.

9. The method of manufacturing a medical agent according to Claim 5, wherein said solid target is prepared by a method comprising the steps of:
pressing the drug powder and simultaneously heating the drug powder to a temperature immediately below the melting point of the drug powder so as to melt a part of said drug powder; and
rapidly cooling and solidifying the molten drug thereby to prepare the solid target.

10. A medical agent manufacturing apparatus comprising:
a solid target composed of drug powder;
a laser generating equipment for irradiating laser beam to said solid target so that intermolecular bonds of drug components are broken and the drug components are released from said target;
a drug container for generating nanoparticles, having an average diameter of 100 nm or less, from said released drug components and for depositing said nanoparticles onto a surface of excipient particles; and
a vacuum chamber for accommodating the solid target and said drug container.

11. The medical agent manufacturing apparatus according to Claim 10, further comprising a vibrating device for applying vibration to said excipient particles to which said nanoparticles generated from said solid target are deposited, so that said nanoparticles deposited to said excipient particles are fluidized.

12. The medical agent manufacturing apparatus according to Claim 10, wherein said solid target contains protein.
